Europäisches Patentamt

European Patent Office

Office européen des brevets

(11) Publication number: **0 386 275 A2**

# EUROPEAN PATENT APPLICATION

(21) Application number: **89103738.4**

(22) Date of filing: **03.03.89**

(51) Int. Cl.5: **C12P 21/00, A61K 37/36, A61K 39/215, C07K 7/00**

(30) Priority: **17.03.88 US 169862**

(43) Date of publication of application:
**12.09.90 Bulletin 90/37**

(84) Designated Contracting States:
**AT BE CH DE ES FR GB GR IT LI LU NL SE**

(71) Applicant: **SYNBIOTICS CORPORATION**
**11011 Via Frontera**
**San Diego California 92127(US)**

(72) Inventor: **Vodian, Morton A.**
**3626 Ryan**
**Escondido California 92025(US)**

(74) Representative: **Sheard, Andrew Gregory et al**
**Kilburn & Strode 30, John Street**
**London WC1N 2DD(GB)**

(54) **Anti-idiotype intermediate method of making binding site mimics.**

(57) A method of determining the structural conformation of the specific binding site of a ligand by making an anti-idiotype having a CDR which is an internal image of the specific binding site of interest, identifying the nucleotide sequence of the DNA encoding the CDR and generating a conformational model from the corresponding amino acid. A method is provided to synthesize a binding site mimic having conformational fidelity to the specific binding site.

EP 0 386 275 A2

# ANTI-IDIOTYPE INTERMEDIATE METHOD OF MAKING BINDING SITE MIMICS

This invention relates to anti-idiotype antibodies, and more specifically to an anti-idiotype antibody intermediate method to determine the conformational structure of ligands or their specific binding partners.

## BACKGROUND OF THE INVENTION

The ability to understand basic metabolic processes has been enormously enhanced by new techniques of molecular biology. Many processes are now recognized to be mediated on the molecular level by the specific binding of one complex molecule, or ligand, to another complementary ligand, resulting in a change in activity of one or both members of the ligand pair. Such ligand-ligand interactions are highly specific and result from the complementary structural conformation of the binding sites of the ligand pairs. Illustrative of such ligand-ligand pairs are antigen-antibody, ligand-receptor, enzyme-substrate, enzyme-cofactor, enzyme-inhibitor, DNA-DNA, DNA-RNA, and a host of molecules including hormones, drugs, viruses and antigens which may bind to corresponding receptors on the cell surface.

Innovative techniques such as X-ray crystallography and computer modeling have provided insight to the structural conformation of some ligands. Other ligands, particularly receptors, are not susceptible to such techniques because for example, they cannot be purified in sufficient quantity. Others cannot be crystallized because of their chemical composition.

Antibodies provide one example of a member of a specific binding pair. Antibodies are complex glycoproteins which are produced by certain cells of the immune system in response to stimulation by materials called antigens, which the host organism recognizes as foreign. A mammal is capable of producing some millions of different species of antibodies, each species of which is capable of recognizing and binding to a specific site on an antigen, termed an epitope. In response to the presence of a particular antigen, antibodies are produced having a binding site, termed the antibody binding site or CDR, which is conformationally complementary to an epitope of the antigen. The variable region of an antibody typically comprises a small portion of the total antibody molecule.

Antibodies themselves can function as antigens. For example an animal can be immunized with a particular antibody, resulting in the production of antibodies specific to epitopes of the immunizing antibody. Antibodies specific to the CDR of the immunizing antibody are termed anti-idiotype antibodies or anti-idiotypes. Thus, the CDR may be capable of binding to both an epitope of an antigen and the variable region of an anti-idiotype. Because both of these specific binding reactions are based on the conformational complementarity of the binding pairs, the CDR of the anti-idiotype may, in fact, be conformationally similar to the antigenic epitope. Such conformationally equivalent CDRs are termed internal images.

Recently, attempts have been made to employ internal image-bearing anti-idiotypes in place of antigens. For example, in U.S. Patent No. 4,490,358 there are described methods of using an anti-idiotype as a synthetic vaccine in place of a killed or attenuated pathogen. Such synthetic vaccines may be advantageous in that they obviate the risks associated with introducing incompletely killed or attenuated pathogens into a susceptible animal. However, the use of such anti-idiotypes has certain disadvantages associated with the presence of the remaining non-CDR portion of the antibody. For example, the non-binding portion, which may account for over 95% of the protein molecule, may itself carry epitopes recognized by the recipient as foreign, and thereby capable of producing an immune response leading eventually to the clearing of the antibody from the host. Alternatively, if not recognized as foreign, the anti-idiotype may be perceived by the host as an immune system regulatory entity, thereby evoking either an enhancing or inhibiting effect depending on the concentration of the anti-idiotype introduced. Antibody fragments (Fabs) produced by cleavage with proteolytic enzymes such as trypsin and papain are known, but these also contain a substantial proportion of the non-binding region.

Thus there exists a need for a method of determining the structural conformation of the specific binding site of a ligand pair and preparing a synthetic molecules having such a structural conformation, or a variant thereof. While anti-idiotype internal images provide such a structural conformation, there exists a need for such an internal image substantially free of extraneous matter. The present invention fulfills such needs and provides related advantages as well.

## SUMMARY OF THE INVENTION

The present invention provides method of determining the structural conformation of the specific binding site of a ligand by making an anti-idiotype having a CDR which is an internal image of the specific binding site of interest, identifying the nucleotide sequence of the DNA encoding the CDR and generating a conformational model from the corresponding amino acid sequence. In another aspect, a method is provided to synthesize a binding site mimic having conformational fidelity to the specific binding site. Such a binding site mimic can be used to screen potential specific binding partners of the original ligand for activity. In another aspect, the binding site mimic can itself be used as a vaccine or can be conjugated to an imaging agent or a toxin. Moreover, the binding site mimic can be used therapeutically as, for example, a hormone or drug analogue to bind to and thereby activate or inactivate the corresponding specific binding partner. In another aspect, the determination of the conformational structure of the CDR can be used to generate variant molecules having altered specific binding capacity.

## BRIEF DESCRIPTION OF THE DRAWING

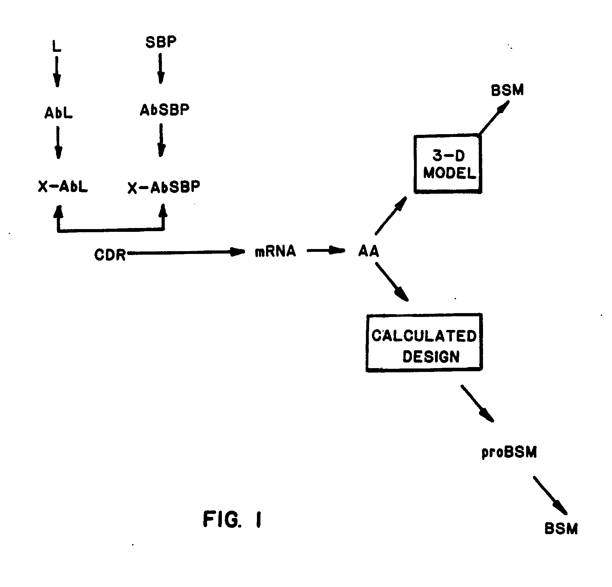

FIGURE 1 shows, by means of a flow diagram, the method and product of the present invention.

## DETAILED DESCRIPTION OF THE INVENTION

The invention relates to methods of determining and synthetically reproducing the structural conformation of the specific binding site, of a ligand or its specific binding partner. Such a method has great advantages in allowing the synthesis of specific peptides or other molecules, able to bind, and thereby activate or inactivate the complementary ligand. The synthetic peptide may be identical to the binding site of the original ligand, or may be altered so as to have increased or decreased binding affinity.

## DEFINITIONS

As used herein, the term "ligand" refers to any molecule which specifically recognizes and binds to another molecule through specific binding site. The term refers to either partner of a specific binding pair. Such binding pairs may include, but are not limited to antigen-antibody, ligand-receptor, enzyme-substrate, enzyme-cofactor, enzyme-inhibitor, DNA-DNA, DNA-RNA, and other molecules which bind to corresponding receptors on the cell surface, each of which may be referred to as a ligand-ligand pair. Herein, ligand-ligand pairs will generally be referred to as ligand and its specific binding partner.

As used herein, the term "binding site mimic" refers to synthetic peptides or other molecules which retain the specificity of the ligand from which they are derived, and have the same or altered affinity for their specific binding partner. Because the binding specificity of a ligand derives from the structural conformation of its specific binding site, the conformation of the specific binding site of the ligand and the binding site mimic will be substantially equivalent. The binding site mimic is intended to be substantially free of structures extraneous to the binding site itself, but may include structures necessary to maintain the conformation of the binding site.

As used herein, the term "structural conformation" or "conformation" refers to the three dimensional shape and topology of a molecule, including the charge distribution across the three dimensional shape.

As used herein, the term "complementary" or "conformationally complementary" refers to ligand binding sites which have opposite conformations such that they may specifically, selectively and preferentially bind to each other. Such binding may involve either covalent or non-covalent bonds. Complementarity reflects what may be colloquially termed a "lock-and-key" or "mold-and-cast" relationship.

As used herein, "anti-idiotype" or "anti-idiotypic antibody" refers to an antibody raised against a first antibody which specifically binds to the CDR of the first antibody.

As used herein, the term "CDR" refers to the antibody binding site of an antibody and is that portion of the antibody which specifically binds to the recognized epitope. It is encoded by a variable region of the nucleic acid which encodes the antibody.

As used herein, the term "internal image" refers to the CDR of an anti-idiotype antibody, raised against

3

a first antibody, wherein the CDR has a structural conformation substantially equivalent to the specific binding site recognized by the first antibody.

As used herein, the term "specific binding site" refers to the portion of a ligand which specifically binds to the ligand's specific binding partner. It is encoded by the "ligand binding site nucleic acid", which is a portion of the nucleic acid encoding the ligand.

The invention relates to an anti-idiotypic antibody intermediate method for representation and duplication of a specific binding site. Anti-idiotypic antibodies to a ligand and to its specific binding partner are raised. The monoclonal anti-idiotype antibodies that maintain three-dimensional fidelity to either ligand or specific binding partner can then be used to produce synthetic peptides or other molecules which mimic the specific binding site. Binding site mimics (BSM) can be produced by analysis of the specific antibody messenger RNA. Identification and sequencing of the variable regions yields information that can be used by appropriate computer protein modeling systems to lead to the synthesis of BSM's. Alternately, after analysis of the antibody binding region (CDR) peptide segments can be synthesized and tested as BSM's. Through amino acid addition, deletion or modification of the proBSM, a specific BSM can be synthesized.

The invention is based on the concept of utilizing the information contained in anti-idiotype antibodies to generate models of the conformational structure of the specific binding site of a ligand. Such models may be further utilized to construct molecules having structural conformations equivalent or substantially equivalent to the specific binding site of the ligand (binding site mimics, or pro-binding site mimics). Such binding site mimics have a variety of utilities, such as the screening of potential specific binding partners for binding affinity, in vitro diagnostics such as competitive immunoassays, in vivo diagnostics such as radio-imaging, in vivo prophylaxis such as vaccines, and in vivo therapeutics such as their use as hormone or drug analogues to activate or inactivate a particular specific binding partner. Moreover, such binding specific mimics can be modified so as to alter their binding affinity for a specific binding partner, as desired.

Figure 1 illustrates, by means of a flow chart, the basic procedures for obtaining and utilizing the information of an anti-idiotype antibody. Briefly, a ligand-ligand pair, herein termed a ligand-specific binding partner pair (L:SBP), is selected, and antibodies raised to both the ligand and the specific binding pair (AbL and AbSBP, respectively). Methods of producing antibodies are well known in the art. Preferably, the antibodies raised are monoclonals. The general methodology for making monoclonal antibodies by hybridomas is well known. See, for example, Kohler and Milstein (1975) Nature 256:495.

Immortal, antibody-producing cell lines can also be created by techniques other than fusion, such as direct transformation of B lymphocytes with oncogenic DNA, or transfection with Epstein-Barr virus. See, for example, Schreier et al., HYBRIDOMA TECHNIQUES (1980); Hammerling et all., MONOCLONAL ANTIBODIES AND T-CELL HYBRIDOMAS (1981); Kennett et al., MONOCLONAL ANTIBODIES (1980). Preferably, the hybridomas are formed by fusing mouse lymphocytes with mouse myeloma cells, although cells from other mammals, such as rats, dogs, rabbits or humans may alternatively be used.

Alternatively, but less desirably, polyclonal antibodies may be used. In this case, natural or synthetic purified ligand and purified specific binding partner is used to immunize a selected mammal (e.g. mouse, rabbit, goat, horse, etc.), and serum from the immunized animal later collected and treated according to known procedures. Compositions containing polyclonal antibodies to antigens in addition to the desired antigen can be made substantially free of the undesired antibodies by passing the composition through a column to which the desired antigen has been bound. After washing, polyclonal antibodies to the bound antigen are eluted from the column.

AbL and AbSBP are in turn used to raise monoclonal anti-idiotype antibodies, X-AbL and X-AbSBP, again by methods well known in the art as described above. Regardless of whether both generations of antibodies are monoclonal, or whether the first antibodies are polyclonals, both the antibodies and the anti-idiotype antibodies are raised in the same species.

In order to identify those anti-idiotype antibodies carrying a CDR which is an internal image of the L or SBP, X-AbL and X-AbSBP are screened against each other to determine those which are able to bind. Since both antibodies are of the same origin and thus cannot bind to each other except through complementary CDRs, those which do so bind must have CDRs which maintain conformational fidelity to the specific binding site of the original ligand or specific binding partner.

Next, the nucleotide sequence encoding the CDR of the X-AbL or X-AbSBP is determined. messenger RNA from the hybridoma secreting the anti-idiotype antibodies is isolated and sequenced by methods well known in the art. This sequence is then compared to known immunoglobulin libraries. Mouse immunoglobulins are well characterized and libraries are available. See, for example, Genebank, available from The National Institute of Health, Bethesda, MD. Several software packages are available which can compare a sequence to known libraries so as to determine those sequences which differ, including, for example, DNA Inspector II + (Textco, Princeton, NH): IBI/Pustell (International Biotechnologies, Inc., New Haven, CT); PC Gene (Intelligenetics, Mountain View, CA); Genepro (Riverside Scientific Enterprises, Seattle, WA); and Microgenie (BioRad, Richmond, CA). In this case, the variable region encoding the CDR will differ from the predominant sequences. From this nucleotide sequence, the amino acid sequence of the CDR itself can be established, based on the well known genetic code.

Various computer software programs are available which can generate a calculated design of the probable conformation of a peptide from its amino acid sequence: for example, Hopp and Woods, (1983) "A computer program for predicting protein antigenic determinants." Molecular Immunology 20:483; Kyte and Doolittle (1982) "A simple method for displaying the hydropathic character of a protein." J. Molec. Biol. 157:105; and Chou and Fasman (1978) "Empirical predictions of protein conformation." Ann. Rev. Biol. 47:251; which are incorporated herein by reference. From such analysis, the hydrophilic regions of the peptide, those most likely to lie on the surface, can be determined.

These hydrophilic sequences, or regions otherwise identified as lying on the surface when the antibody molecule assumes a tertiary structure, can then be joined in a linear array; such a linear array is unlikely, however, to assume a three dimensional configuration so as to provide binding activity resembling the original ligand or specific binding partner. However, methods are known for determining appropriate bridging sequences between peptide fragments which will orient and maintain the fragments in the correct conformational configuration so as to achieve activity. For example, U.S. Patent No. 4, 704, 692, entitled COMPUTER BASED SYSTEM AND METHOD FOR DETERMINING AN DISPLAYING POSSIBLE CHEMICAL STRUCTURES FOR CONVERTING DOUBLE- OR MULTIPLE-CHAIN POLYPEPTIDES TO SINGLE-CHAIN POLYPEPTIDES, which is incorporated herein by reference in its entirety, discloses methods for determining appropriate linkers to bridge the gap between fragments derived from separate chains of a polypeptide.

A peptide composed of a linear array of surface fragments, which may be joined by rudimentary bridging sequences, is herein termed a pro-binding site mimic (pro- BSM). Such a pro-BSM may have structural conformation suggestive of that of the ligand or specific binding partner which is mimicked. The amino acid sequence of the pro-BSM may then be systematically modified in order to achieve greater conformational fidelity. For example, computer assisted methods such as those disclosed in U.S. Patent No. 4,704,692, supra, may be repeated in order to fine-tune the structure provided by the linkers. Alternatively, systematic amino acid substitution may be utilized to produce synthetic pro-BSM's which differ by one amino acid. These modified pro-BSM's are then screened for binding affinity to the specific binding partner of the mimicked ligand, an anti-idiotype carrying an internal image of such specific binding partner, or, indeed, a binding specific mimic or such specific binding partner.

A method for synthesizing monomers which are topographically equivalent to a ligand which is complementary to a receptor of interest is disclosed in World Patent No. 86/06487, entitled METHOD FOR DETERMINING MIMOTOPES. Disclosed therein is a method of synthesizing dimers composed of random combinations of monomers and screening these for binding affinity against a receptor of interest. Dimers exhibiting activity then condensed to an additional random monomer to form an array of trimers which are again screened for activity.

The present invention is distinguished by utilizing proBSM's or BSM's which are preselected to have conformational fidelity to the desired specific binding site of the ligand of interest. Particular amino acid positions can be chosen for modification, either by addition, deletion or substitution of the amino acid. Such sites can be determined randomly, or can be selected on the basis of their presumed importance to the conformational structure of the peptide. A vast array of substituents can be utilized to generate variants. For example, at any one site, the amino acid of the pro-BSM can be substituted with a substituent selected from, for example, D-amino acids, L- amino acids, or any other chemical moiety suspected to confer the appropriate characteristics.

Alternatively, in accordance with another embodiment of the present invention, rather than synthesizing pro-BSM variants, computer assisted methods can be used to determine the conformational structure of such substituted pro-BSM's and select desired substitutes.

Moreover, if the ligand of interest is not a peptide, but rather a molecule composed of subunits, the methods of the present invention may also be used to generate variants and to screen for binding affinity, by methods analogous to those described above.

While it is assumed that, normally, increased conformational fidelity will be desired, in fact, the methods of the present invention may also be advantageously used to generate BSM's having altered binding affinity or decreased binding activity from that of the mimicked ligand. Such altered mimics may have utility, for example, where it is desirable to bind to but not activate the receptor.

In another embodiment of the invention, the entire amino acid sequence of the anti-idiotype, or a substantial proportion thereof may be subjected to computer assisted scrutiny g"computer modeling" in order to determine from the tertiary structure of the whole antibody molecule the topography and binding characteristics of the binding site itself. An example of such a computer assisted program can be found in the GROMOS molecular dynamics package (Agvist, et al., (1985) J. Molec. Biol. 183:461) or FRODO: A Molecular Graphics Program (Jones, T.A. COMPUTATIONAL CRYSTALLOGRAPHY 303, Clarendon, Oxford (1982)) which are incorporated herein by reference.

These methods will predict the secondary and tertiary structures of the interacting heavy and light chains of the antibody and the specific binding site. Moreover, this information itself permits the derivation of an amino acid sequence, of three to twenty or more residues, which is conformationally structurally equivalent to the specific binding site (BSM). In the case where such a conformationally structurally equivalent sequence is generated for an anti-idiotype antibody, the sequence can be synthesized and tested for binding to its corresponding idiotypic antibody (or the appropriate ligand or its mimic). The sequence can again serve as a starting point for amino acid additions, deletions, or substitutions. in this manner, an optimal sequence can be produced which duplicates (or modifies) the structure of the anti-idiotypic CDR.

Synthetic peptides (or other chemical molecules) which are conformationally equivalent to the specific binding site of a ligand of interest are termed binding site mimics (BSM's). BSM's which are modified so as to have a specific variant binding activity are termed modified BSM's.

BSM's have a variety of utilities. They can, for example, be used as vaccines, thereby avoiding the problems associated with vaccinating with either attenuated pathogens or anti-idiotypic antibodies (or Fabs). Moreover, their specific binding capacity can be utilized in a manner analogous to that employed with whole antibodies for in vivo diagnostics, such as radio-imaging, or in vivo therapeutics such as radio immunoconjugates or immunotoxins, according to methods described in the art for antibodies. See, for example, U.S. Patent No. 4,331,47 ; 4,460,559; 4,460,561; and 4,624,846 which are incorporated herein by reference.

Additionally, these BSM's may be used as drug or hormone analogues so as to activate or inactivate the corresponding receptor. Moreover, they may be used in various immunoassays,1 such as, for example, a competitive immunoassay.

The following examples are intended to illustrate but not limit the invention. While they are typical of those that might be used, other alternative procedures known to those skilled in the art may be employed in their place without departing from the scopes of the invention.

EXAMPLE I

PREPARATION OF ANTI-IDIOTYPE ANTIBODIES TO LIGAND RECEPTOR

A. Porcine Somatotropin

Porcine somatotropin (pST) is a natural growth hormone and may be isolated and purified by the method of Dellacha and Sonnenberg (1964) J. Biol. Chem. 239:1515-1520, which is incorporated herein by reference. Specific pST receptors and their purification have been described in various tissues of several species, Gorden et al., (1973) Pharmacol. Rev. 25:179-190, which is incorporated herein by reference.

Plasma membrane pST receptors are prepared from the livers of pregnant rabbits. in the preferred method, all work is carried out at 4 degrees C. A liver from a third trimester pregnant rabbit is homogenized in 0.3M sucrose using a Polytron PT 10 (Brinkman Instruments, Westburg, NY) homogenizer. The homogenate is passed through a cheesecloth filter to remove cellular debris. The supernatant is centrifuged at 15,000 X G for 20 minutes and the resulting supernatant is centrifuged at 130,000 X G for 45 minutes. This pellet contains the major portion of plasma membranes. The pellet is suspended in 25 mM Tris-HCl buffer containing 10 mM MgCl2. The same procedure can be used to isolate rat liver receptors. PST receptor is coupled to keyhole limpet hemocyanin (KLH) by standard techniques so as to enhance its immunogenicity. Ten mice are immunized by i.p. injection of a mixture of 50 ug of pST receptor mixed with an equal volume of complete Freund's adjuvant. The mice are rested for 4-8 weeks and then boosted with the receptor preparation as before except for the use of incomplete Freund's adjuvant.

The immune response of each donor is followed during the inoculation period in order to determine the best responder. The immune response is monitored by measuring antibody titers at alternating two week intervals until a suitable titer is reached. Serum samples are diluted in 25 Tris-Hcl buffer containing 10 mM MgCl2 and 0.1% BSA, pH 7.4, to make serum dilutions of 1:5, 1:10, 1:100, and 1:1000 (final assay dilutions are 1:25, 1:50, 1:500, and 1:5000 respectively). 100 ul of diluted serum is combined with 100 ul of 125-I-pST (20,000 cpm in Tris buffer), 200 ul of receptor (250 ug receptor protein) and Tris buffer to a final volume of 500 ul. Non-specific binding (NSB) controls for the mouse bleeds include tubes containing 100 ul (250 ug receptor protein) of pST plus the diluted mouse bleeds. Assay controls include tubes containing normal mouse serum in place of the mouse bleeds with (NSB) or without added pST. The tubes are incubated overnight at 4 degrees C. The reaction is stopped by adding 2 ml of cold water and centrifuging for 20 minutes at 3000 rpm, 4 degrees C. The supernatants are decanted and discarded. The pellets are counted in the gamma counter for 1 minutes. If antibody is present in the serum, the amount of 125-I-pST bound to receptor will be reduced.

When the antibody is "sufficiently high", i.e. antibody titer does not increase with additional boost, the mouse with the highest antibody titer receives one addition i.p. injection of pST receptor-KLH (for other ligands, the injection if i.v.). Three days later the spleen is removed and fused with myeloma cells and is described as follows.

The best responder from each group was identified and its spleen removed. Each spleen was then placed into buffer solution and converted into a suspension of single cells by means of agitation. Cell counts of lymphoid cells in the suspensions were taken. The suspension of spleen cells were subsequently employed in a fusion step with myeloma cells.

The myeloma cells should be selectable, i.e. the growth of the myeloma cells in culture should be dependent upon a factor supplied by the culture medium. However, upon fusion with an antibody expressing cell, the resultant cellular fusion product loses its dependence upon this factor. Hence, the cellular fusion product can grow in culture in the absence of this factor. Preferred myeloma cell lines include $P_3X63$-Agul and P3X63-Ag8.853, each of which were supplied by American Type Tissue Collection. Directly prior to the fusion step, the myeloma cell line was placed into its "S" growth phase.

During the fusion step a cell mixture was made by combining a sample of the myeloma cells with both a sample of the suspension of spleen lymphoid cells from the best responder mouse with respect to pST. Best results were found using a cell mixture having a cellular composition of 1:2, i.e. one part myeloma cells, two parts spleen lymphoid cells.

In order to induce fusion, the above cell mixture was combined with polyethylene glycol (PEG 1000) under standard conditions to form a 50% solution in culture medium. After one or two minutes, the resultant cell mixture was diluted with medium, i.e. Dulbecco's minimal essential medium (DMEM), 10% fetal bovine serum, hypoxanthine, aminopterin, and thymidine. The dilution was calculated to yield a cell mixture with approximately $1-1.5 \times 10(6)$ cells per milliliter. Aliquots of 0.2 milliliters of the diluted cell mixture were then pipetted into microtiter plates, preferably 96-well plates. The wells of the microtiter plates included a feeder layer of mouse thymocytes. The resultant fusion products were then incubated in the microtiter plates for 10-12 days. Supernatants from hybridomas are screened for antibody production, antibody producing cells are diluted and screened (expansion screen), diluted for monoclonal antibody production, screened, expanded again and screened again. Selected monoclonal antibody producing cells are grown and injected into mice for ascites antibody production.

Antibody screening is based on the competition by cell supernatants (presumably containing pST receptor antibody) and 125-I-pST for binding sites on the pST receptor. The assay diluent used in this assay is 25 mM Tris-HCl buffer containing 10 mM MgCl2 and 0.1% BSA, pH 7.4. 100 ul of cell supernatants are transferred to 12 x 75 mm glass tubes. 100 ul of 125-I-pST and 100 ul of pST receptor (250 ug) from livers of pregnant rabbits are added. Non-specific binding controls contain 100 ul of pST (2.5 ug) in place of cell supernatants and maximum binding controls contain Tris buffer but no cell supernatant or additional pST. Supernatant containing an irrelevant antibody is assayed as a quality control. The tubes are incubated overnight at 4 degrees C. The reaction is stopped by adding 2 ml of cold water and centrifuging for 20 minutes at 3000 rpm, 4 degrees C. The supernatants are decanted and discarded. The pellets are counted for 1 minute in the gamma counter. If antibody to pST receptor is present in the cell supernatant, the amount of 125-I=pST bound to receptor will be reduced. After production of the appropriate ascitic fluid, purified antibody is produced by the following method. Saturated ammonium sulfate was added dropwise with continuous stirring to an equal volume of ascites fluid in a Beckman 5/8 x 3 inch polycarbonate tube, stirred for an additional 15 minutes at room temperature, and then centrifuged for 15 minutes at 10,000 rpm, 4 degrees C. The supernatant was discarded. The pellet was suspended in 1/2 volume of PBS and an equal volume of ammonium sulfate added dropwise, stirred for an additional 15 minutes and centrifuged as above. The pellet was resuspended in 1/2 volume of PBS and dialyzed against 1 liter of 0.01 M phosphate buffer containing 0.2 M NaCl, pH 7.4, overnight with one change of dialysis buffer. A 10 ml disposable pipette was packed with 10 ml of DEAE (250 mm hydrostatic pressure). The column was washed with 0.5M phosphate buffer, pH 7.0, then with 0.5M acetate buffer, pH 6.0, until the eluate was pH 6.0. The column was washed with 0.5M phosphate buffer, pH 7.0, until the eluate was pH 7.0 and then washed with several volumes of 0.05M phosphatase buffer, pH 7.0. The column outflow tube was attached to a UV monitor (280 nm) and the monitor outflow tube to a collection vessel. The dialyzed sample was transferred to the column and eluted with 0.05 M phosphate buffer, pH 7.0. Collection vessels were changed when the protein begins passing through the UV monitor and stopped collecting eluate at the end of the protein peak. The protein assay was based on an assay protocol using BCA protein assay reagents from Pierce Chemical Company, Rockford, IL. Standards were made up from a 2 mg/ml stock of bovine albumin powder in 0.001 M NaHCO3. A standard protein concentration range of 0-250 ug/ml in a total volume of 2.0 ml made up in the same buffer as the test material in each assay. 0.1 ml of each standard or test sample was pipetted into 13 x 100 mm tubes and 2.0 ml BCA working reagent (50 parts Reagent A plus 1 part Reagent 2) was added and mixed. The tubes were incubated at 60 degrees C for 30 minutes and then cooled to room temperature. The absorbency was read at 562 nm against a water blank. The protein values of test samples were determined by comparing the O.D. of the test sample with the bovine albumin standard curve. Purified pST receptor antibody was used to immunize mice to produce anti-idiotypic antibodies to the pST receptor. The immunization schedule and the fusion schedules were the same as above for pST. The screening methods for both the mouse bleed titers and monoclonal anti-idiotypic antibody to pST receptor are described below. The assay is based on the fact that the pST receptor anti-idiotypic antibody reacts in the same manner as pST receptor and therefore should have a specific binding site for pST. The assay diluent was 25 mM Tris-HCl buffer containing 10 mM MgCl1 and 0.1% BSA, pH 7.4. Mouse bleeds were diluted to 1:5, 1:10, 1:100, and 1:1000 (1:25, 1:50, 1:500, and 1:5000 final assay dilutions). Normal mouse serum was similarly diluted for use as assay controls. 100 ul of diluted test sera and normal sera were transferred to 12 x 75 mm tubes. 100 ul of 125-I-pST (20,000 cpm) and diluent was added (500 ul final volume) and mixed. Non-specific binding control tubes contain 2.5 ug of pSt. An additional set of controls contained 200 ul of receptor (250 ug of rabbit liver receptor protein). The tubes were incubated overnight at 4 degrees C. The reaction was stopped by adding 50 ul of normal bovine serum and 1.5 ml of 16.7% polyethylene glycol (12.5% final PEG) in veronal buffer, pH 8.6. The tubes were incubated for 10 minutes at 4 degrees C and then centrifuged at 3000 rpm for 20 minutes at 4 degrees C. The supernatants were decanted and discarded. The radioactivity of the pellets was then determined.

The anti-idiotypic antibody screening assay was similar to that used to screen mouse bleeds except that the diluent for all materials except the 125-I-pST was cell culture media; that diluent being Tris-HCl buffer with MgCL2 and BSA as described previously. 100 ul of supernatants from fusions, fusion screens, monoclonal antibody screens, or monoclonal antibody expansions were combined with 100 ul of 125-I-Gh (20,000 cpm) in 12 x 25 mm tubes. Controls include 50 ul of theophylline and 50 ul of pST receptor (250 ug of liver plasma membrane protein) plus 125-I-pST, 50 ul (2.5 ug) pST plus receptor and 125-I-pST (NSB), and receptor plus 125-I-Gh, all at a final volume of 200 ul. The tubes were incubated overnight at 4 degrees C. The reaction was terminated by adding 50 ul of normal bovine serum and 1.5 ml of 16.6% polyethylene glycol in veronal buffer, pH 8.6. The tubes were centrifuged at 3500 rpm for 15 minutes at 4 degrees C and the supernatants were decanted and discarded. Radioactivity of the pellets are determined by use of a gamma counter.

## B. Feline Infectious Peritonitis

FIP is a feline enteric corona virus originally described by Robinson et al., (1971) J. Amer. Vet. Med. Ass 158:981. The virus can be produced by infecting monolayer cellular cultures of the fcwf cell line obtainable from the American Type Culture Collection (Bethesda, MD). it is composed of at least three major proteins E1, E2 and a nucleocapsid protein. E1 and E2 proteins form a peplomer structure which protrudes from the surface of the virus and serves as the specific element which interacts with specific receptors of potentially infective host cells. Specific cell receptors for FIP have not been described; however, they have e been described for other viruses, specifically, mouse rheovirus, Gaulton et al., (1986) J. Immunol. 137:2930.

For the generation of anti-idiotypic antibodies to FIP receptors Balb/c Ann mice were given a primary injection I.P. with 1.0 x 10(7) live, whole uninfected fcwf cells without adjuvant and rested 4-6 weeks. A bleed-boost schedule was continued every 2-3 weeks and serum titers were checked one week after each boost by the following receptor assay. Generation of putative anti-receptor monoclonal antibodies were assayed by first mixing serum samples in serial dilutions with uninfected fcwf cells in 96-well plates. The mixture was incubated at 37 degrees C for 2 hours, after which 25 PFU of infectious FIPV is added per well and plates were incubated at 37 degrees C for 30-48 hours. When positive control (virus, but not antibody) has reached total cytopathic effect, all wells were then fixed with 25% formalin and stained with crystal violet. Plates were examined microscopically. Positive wells showed a healthy confluent monolayer of cells which take up stain while negative wells did not take up stain and were literally blown away. A negative control includes preincubation of cells with IVFP12 522.13, a monoclonal generated at Synbiotics that has been confirmed repeatedly to offer protection against FIP infections in vitro. This antibody binds to the surface of fcwf cells as demonstrated by FACS analysis. When serum titers reached approximately 1:51,000 the mouse was sacrificed and the spleen fused according to the protocol described above for pST. Fusion was screened once hybridoma growth was well established (10-14 days) by receptor assay described above. Positive mothers were expanded and cloned as soon as possible after expansion by limiting dilution in complete media with HAT and thymocytes. Cloning used two 96-well plates per mother at cell concentrations of 100 to 200 cells per plate. After growth was established, SNF from all monoclonals was assayed by receptor assay. Positives were confirmed to be monoclonal by microscopic examination and further characterized by isotyping and Western blot specificity. The best candidate from each mother was expanded and injected into mice for ascites production and a backup clone was frozen. Each clone was injected into 5 Balb/c Ann mice at a concentration of 3.0 x 10(6) cells per mouse in 0.50 ml complete media. For the preparation of Ab1 for immunization, ascites was freon treated and purified by PEG precipitation for IgM or ion exchange for IgG. Candidates were chosen by such parameters as receptor assay results, isotype, and Western blot specificity. Purified antibodies were then conjugated to KLH using standard techniques and used as immunogen for Ab2 production. The protocol for immunization is as follows:

| Five Mice | | | Five Mice | |
|---|---|---|---|---|
| | 1) | 50 ug Mab-KLH in CFA, i.p. | 1) | 50 ug Mab-KLH in CFA, i.p. |
| | 2) | Rest 4 weeks | 2) | Rest 8 weeks. |
| | 3) | Boost #1. 50 ug Mab in IFA, i.p. | 3 - 10) | Same as previous groups. |
| | 4) | Rest 1 week. | | |
| | 5) | Blood #1. | | |
| | 6) | Rest 1 week. | | |
| | 7) | Boost #2. 50 ug Mab-KLH in 1 x PBS, i.p. | | |
| | 8) | Rest 1 week. | | |
| | 9) | Bleed #2. | | |
| | 10) | If titer indicates, make fusion arrangements w/25 ug Mab-KLH in 1 x PBS I.V. boost or rest for an additional week and boost a third time as per (7.). | | |

Detection of serum anti-idiotypic antibody titers was monitored by ELISA as follows, using standard incubation times and wash protocols. Ab1 was used as the solid phase capture antibody. Following blocking step, test serum was added and incubated. After washing, HRP conjugated Ab1 is introduced as the detect antibody. After incubation and washing, ABTS 2, 2-Azino-di-(3-Ethylbenzthiazoline Sulfonic Acid) substrate was added. Plates were read on ELISA reader (MR600 Microplate Reader, Dynatech Labs, Inc., Alexandria, WV), and the titer determined by O.D. reading. When titers showed a significant increase, the mouse with the highest titer received a final I.V. boost and was fused three days later. The fusion, fusion screen and cloning procedures were identical to those described above. Clones were further characterized by inhibition assays where Ab2 obstructs Ab1 recognition of antigenic determinants. Inhibiting Ab2's were expanded and injected into mice for the ultimate test of anti-idiotype determination - the production of Ab3 that resembles A1.

EXAMPLE II

## ANTI-IDIOTYPE ANTIBODIES TO LIGAND

A. pST

Purified pST was purchased from International Minerals and Chemical Corporation, Chicago, Illinois. PST was coupled to keyhole limpet hemocyanin (KLH) by standard techniques so as to enhance its immunogenicity. Ten mice were immunized by i.p. injection of a mixture of 50 ug of pST receptor mixed with an equal volume of complete Freund's adjuvant. The mice are rested for 4-8 weeks and then boosted with the receptor preparation as before except for the use of incomplete Freund's adjuvant.

The immune response of each donor was followed during the inoculation period in order to determine the best responder. The immune response was monitored by measuring antibody titers at alternating 2 week intervals until a suitable titer is reached. For testing antibody titers, pST was diluted 1:1000 in carbonate buffer, pH 9.6 and added to microtiter plates. The pST was allowed to bind overnight and the excess removed. The plates were washed and irrelevant binding sites blocked with PBS containing 0.5% BSA and 0.5% Tween for 15-30 minutes. After washing the plates, serum titers (serial dilutions from 1:50 to 1:102,400) were added to the wells and incubated for 30 minutes. After washing, goat-anti-mouse antibody was added and the plates incubated for 30 minutes. The plates were washed and HRP substrate added. The plates were read after 30 minutes and positive wells determined.

When the antibody was "sufficiently high", i.e. antibody titer does not increase with additional boost, the mouse with the highest antibody titer received one additional i.p. injection of pST-KLH (for other ligands, the injection is i.v.). Three days later the spleen is removed and fused as above and described as above. Supernatants from hybridomas were screened for antibody production, antibody producing cells were diluted and screened (expansion screen), diluted for monoclonal antibody production, screened, expanded again and screened again. Selected monoclonal antibody producing cells were grown and injected into mice for ascites antibody production.

For the antibody screening assay, veronal buffer, pH 8.6 was prepared by mixing 9.2 gm of sodium barbital and 1.5 gm. of barbital plus 0.1 gm of sodium azide in water to make 1 liter. The diluent consisted of 0.2 gm. of BSA, 1.0 ml of normal mouse serum and veronal buffer to make 100 ml. The pH was then adjusted to pH 8.6. Control diluent consisted of DMEM containing 10% fetal bovine serum, containing -2% L-glutamic acid and 1% HAT. 90 ul of cell supernatant were combined with 90 ul of 125-I-pST (10,000 cpm) in 12 x 75 mm tubes. Controls consisted of control antibody plus 90 ul of 125-I-pST. Non-specific binding controls consisted of 90 ul of control diluent plus 90 ul of 125-I-pST. The tubes were incubated overnight at 4 degrees C. The reaction was stopped by adding 50 ul of cold normal bovine serum and 750 ul of 16.6% polyethylene glycol in veronal buffer. The solution was vortexed to mix, incubated for 10 minutes, and centrifuged for 15 minutes at 3500 rpm at 4 degrees C. The supernatants were decanted and discarded and the radioactivity of the pellets determined.

For the purification of antibody for immunization, saturated ammonium sulfate was added dropwise with continuous stirring to an equal volume of ascites fluid in a Beckman 5/8 x 3 inch polycarbonate tube. The solution was stirred for an additional 15 minutes at room temperature and then centrifuged for 15 minutes at 10,000 rpm, 4 degrees C. The supernatant was discarded. The pellet was suspended in 1/2 volume of PBS and an equal volume of ammonium sulfate added dropwise. The solution was stirred for an additional 15 minutes and centrifuged as above. The pellet was resuspended in 1/2 volume of PBS and dialyzed overnight against liter of 0.01 M phosphate buffer containing 01.2 M NaCl, pH 7.4, with one change of dialysis buffer. A 10 ml disposable pipette was packed with 10 ml of DEAE (250 mm hydrostatic pressure). The column was washed with 0.5 M phosphate buffer, pH 7.0, then with 0.5 M acetate buffer, pH 6.0, until the eluate is pH 6.0, then washed with 0.5 M phosphate buffer, pH 7.0, until the eluate was pH 7.0 and then washed with several volumes of 0.05 M phosphatase buffer, pH 7.0. The column outflow tube was attached to a UV monitor (280 nm) and the monitor outflow tube to a collection vessel. The dialyzed sample was transferred to the column and eluted with 0.05 M phosphate buffer, pH 7.0. The collection vessels were changed when protein began passing through the UV monitor and collecting of the eluate stopped at the end of the protein peak.

The protein assay for the purified antibody was based on an assay protocol using BCA protein assay reagents from Pierce Chemical Company. Standards were made up from a 2 mg/ml stock of bovine albumin powder in 0.001 M NaHCO3. A standard protein concentration range of 0 - 250 ug/ml in a total volume of 2.0 ml was made up in the same buffer as the test material in each assay. 0.1 ml of each standard or test sample wa pipetted into 13 x 100 mm tubes and 2.0 ml BCA working reagent (50 parts Reagent A plus 1 part Reagent 2) was added and mixed. The tubes were incubated at 60 degrees C for 30 minutes and then cooled to room temperature. The absorbency was read at 562 nm against a water blank. The protein values of test samples were determined by comparing the O.D. of the test sample with the bovine albumin standard curve.

Purified pST antibody was used to immunize mice to produce anti-idiotypic antibodies to the pST. The immunization schedule and the fusion schedule were the same as above. The screening methods for both the mouse bleed titers and monoclonal anti-idiotypic antibody to pST receptor were as follows. PST antibody diluted 2-30 ug/ml (as determined in advance) was added to microtiter plate wells and allowed to bind overnight at room temperature. The plates were then flicked to remove the liquid and washed. The unbound well binding sites were then blocked for 15-30 minutes at room temperature with PBS containing 0.5% BSA and 0.5% Tween 20. The liquid was again removed by flicking the plate and washing as above. Serum sample dilutions (1:5-1:102, 400) were added to the wells and incubated for 30 minutes. Horseradish peroxidase was conjugated to the same pST antibody as used to originally coat the plate. The plates were flicked and washed and HRP-pST antibody conjugate added. The solution was incubated for 30 minutes, flicked and washed on the plate and 200 ul ABTS substrate was added. The solution was incubated for 30 minutes and then read for absorbency at 405 nm and 490 nm as above.

Two methods were used for anti-idiotypic antibody screening. The first test was the same as described above except that cell supernatants were tested rather than mouse bleeds,. In the second test, pST was diluted 1:1000 and added to all wells of microtiter plates. The plates were incubated overnight at room temperature after which the liquid was removed by flicking and then washing. Unbound well binding sites were blocked by adding PBS containing 0.5% BSA and 0.5% Tween 20 and incubating for 30 minutes. Simultaneously, pST-HRP conjugate was added to the anti-idiotypic antibody supernatants and allowed to incubate for 30 minutes. The plates were flicked and washed and the HRP anti-id mixture added and allowed to incubate for 30 minutes. The plates were flicked and washed and HRP substrate added. This was incubated for 30 minutes and the absorbency read on an ELISA reader as described above. A low absorbency reading indicating that there was competition between pST and anti-idiotypic antibody present in the supernatant. For the preparation of Ab1 for immunization, ascites was freon treated and purified by PEG precipitate for IgM or ion exchange for IgG. Candidates were chosen by assay results, isotype, and Western blot specificity. Purified antibodies were then conjugated to KLH using standard techniques and used as immunogen for Ab2 production. The protocol for immunization is as follows:

| | | | | |
|---|---|---|---|---|
| 1) | 50 ug Mab-KLH in CFA, i.p. | | 1) | 50 ug Mab-KLH in CFA, i.p. |
| 2) | Rest 4 weeks | | 2) | Rest 8 weeks. |
| 3) | Boost #1. 50 ug Mab in IFA, i.p. | | 3 - 10) | Same as previous groups. |
| 4) | Rest 1 week. | | | |
| 5) | Blood #1. | | | |
| 6) | Rest 1 week. | | | |
| 7) | Boost #2. 50 ug Mab-KLH in 1 x PBS, i.p. | | | |
| 8) | Rest 1 week. | | | |
| 9) | Bleed #2. | | | |
| 10) | If titer indicates, make fusion arrangements w/25 ug Mab-KLH in 1 x PBS I.V. boost or rest for an additional week and boost a third time as per (7.). | | | |

Detection of serum anti-idiotypic antibody titers was monitored by ELISA as follows, using normal incubation times and washes between steps. Ab1 was used as the solid phase capture antibody. Following blocking step, test serum was added and incubated. After washing, HRP conjugated Ab1 was introduced as the detect antibody. After incubation and washing, ABTS substrate was added. Plates were read on ELISA reader and the titer determined by O.D. reading. When titers showed significant increase, the mouse with the highest titer received a final I.V. boost and was fused three days later. The fusion, fusion screen and cloning procedures were identical to those described above. CLones were further characterized by inhibition assays where Ab2 obstructs Ab1 recognition of antigenic determinants. Inhibiting Ab2s were expanded and injected into mice for the ultimate test of anti-idiotype determination - the production of Ab3 that resembles Ab1.

## B. FIP

For the generation of anti-idiotypic antibodies to FIP Balb/c Ann mice were given a primary injection i.p. with 5.0 x 10(6) live, whole infected fcwf cells without adjuvant and were rested 4-6 weeks. Mice were boosted with an i.p. injection of 5.0 x 10(6) infected cells. A bleed-boost schedule was continued every 2-3 weeks and serum titers were checked one week after each boost by the following ELISA assay. Normal incubation times and wash steps were observed. A pre-determined amount of FIP infected fcwf cells or purified virus was used as the solid phase capture antibody. After proper blocking with 0.5% BSA in PBS, serum samples were added in serial dilutions and incubated. After washing, HRP conjugated goat-anti-mouse antibody was added and incubated for 30-60 minutes. After washing again, substrate (ABTS) was added and the absorbency was determined. Using uninfected cells as controls, when titers reached approximately 1:51,000, the mouse with the highest titer was fused.

Fusion was screened once hybridoma growth was established (10-14 days). SNF from all wells were tested by the ELISA format outlined above. Positive mother clones were expanded into 24 well plates and retested for titers and for cross-reactivity to related corona viruses such as Feline Enteric Corona Virus (FeLV), Transmissible Gastroenteritis Virus (TGEV), and Canine Corona Virus (CCV) and avirulent strains of FIP. Virus neutralizing assays were also performed at this time. Positive mothers were cloned as soon as possible after expansion by limiting dilution in complete media with HAT and thymocytes. A typical cloning used two 96-well plates pwer mother at cell concentrations of 100 to 200 cells per plate. After growth was established, supernatant fluid (SNF) from all monoclonals were assayed by ELISA assay. Positives were confirmed to be monoclonal by microscopic examination and further characterized by isotyping and Western blot specificity. The best candidate from each mother was expanded and injected into mice for ascites production and a backup clone was frozen. Each clone was injected into 5 Balb/c Ann mice at a concentraiton of 3.0 x 10(6) cells per mouse in 0.50 ml complete media.

The following descriptive flow chart compares generations of antibodies from one approach to antibodies from the second. The terms are abbreviated as follows:

A = FCWF cells
B = anti-id to anti-FIP receptor
C = anti-FIP viral antibodies
D = anti-FIP receptor antibodies
E = FIP viral antigens
F = anti-id to anti-FIP virus

Anti-FIP receptor antibody has been shown to bind specifically to FCWF cells by FACS analysis using a FACS STAR (Becton Dickinson, Mountain View, CA). This interaction is inhibited by anti-ids to anti-receptor antibodies. FIP viral antigens have been demonstrated to react specifically with anti-FIP virual antibodies by Western analysis and ELISA. Furthermore, binding of FIP virus and anti-FIP viral antibodies has been actively inhibited by anti-idiotypes to anti-FIP viral antibodies in Western blot analysis.

In a viral neutralizing assay (incubation of FIP virus plus antibody, and subsequent infection of FCWF cells) anti-idiotype to anti-FIP receptor antibody renders FIP virus non-infectious.

To further demonstrate the identity of anti-idiotype and anti-FIP virus, 125 I = labeled C was competitively inhibited by unlabeled B in RIA for binding to virus. Reciprocally, labeled B is inhibited by unlabeled C.

The most important demonstration is that anti-FIP receptor antibody and anti-idiotype to anti-FIP viral antibodies induce protective immunity to FIP virus in cats. Clinical studies have indicated that a degree of protection is induced by administration of D or F orally (2 mg) in alum on days 0, 14, and 28 with subsequent viral challenge at day 33.

## EXAMPLE III

## DETERMINATION OF THE AMINO ACID SEQUENCE FOR THE VARIABLE REGIONS OF ANTIBODY MOLECULES

A detailed description of the interactions between an antibody and its epitope is necessary to allow an understanding of the way in which antibodies bind to antigenic surfaces presented by foreign molecules.

Ideally this should be done by analysis of crystal structures of antibody-antigen complexes but so far only a few of these are available. An alternative strategy combines molecular modeling with site-directed mutagenesis to generate a model of the antibody-antigen complex. To derive a molecular model it is essential to know the amino acid sequence of the antibody combining site which is formed by the juxtaposition of six hypervariable or CDR regions, three deriving form the light chain (L1, L2, L3) and three from the heavy chain (H1, +H2, H3) antibody. One method used to determine the amino acid sequence of the CDRs is by sequencing the DNA which encodes the CDRs. The DNA sequence is assigned codons and ultimately amino acid sequence.

To determine the DNA sequence which encodes for immunoglobulin the light and heavy chain variable regions from the hybridoma producing the antibody of interest are cloned. The cloning and sequencing of the variable regions is accomplished in the following outlined manner:

1. Isolation of polyA messenger RNA (mRNA) from the hybridoma (this mRNA encodes for immunoglobulin light and heavy chain as well as other housekeeping proteins).

2. Synthesis of cDNA from the isolated mRNA.

3. Cloning of cDNA into a plasmid vector.

4. Screening of the plasmid clones with probes which are specific for light or heavy chain immunoglobulin sequences (this step separates the heavy and light chain sequences from the housekeeping gene sequences).

5. Sequencing of plasmid clones containing immunoglobulin sequences.

6. Translation of immunoglobulin light and heavy chain DNA sequence into codons and then amino acids.

The end result is determination of the amino acid sequence for immunoglobulin light and heavy chains.

1. RNA (consisting of polyA messenger RNA and other types of RNA such as transfer or ribosomal RNA) can be isolated from hybridomas by the lysing of cells in the presence of guanidine thiocyanate (to prevent degradation of RNA) followed by the passing of the total RNA over an oligodT cellulose column which binds polyA messenger RNA but not other types of RNA (transfer and ribosomal RNA). The polyA messenger RNA can be eluted from the oligodT cellulose under appropriate conditions.

2. cDNA synthesis is by the method of Gubler and Hoffman (1983) Gene. 25:263, which is incorporated herein by reference. The isolated polyA mRNA is annealed to a short oligodT primer. This short double strand structure serves as a template for DNA synthesis upon the addition of reverse transcriptase. The reverse transcriptase synthesizes a DNA strand using the mRNA. After the first DNA strand is synthesized, the addition of RNase H causes degradation of the mRNA. To the single stranded DNA is added DNA polymerase I which synthesizes a second DNA strand. The double strand molecule is referred to as copy DNA or cDNA. The cDNA is then cloned into an appropriate plasmid.

3. The double strand DNA from step 2 is mixed with an appropriate plasmid such as puC19 and T4 ligase enzyme. This reaction places the cDNA within a cloning site on puc19. The puc19 plasmid vector has an ampicillin resistance gene which is necessary for transformation of E. coli strain HB101. Once the cDNA is ligated into the puc19 plasmid, this structure is then forced or transfected into HB101 in order to increase the number of copies of the cloned cDNA sequence. The HB101 harboring the cloned DNA is also screened for immunoglobulin sequences in step 4. As indicated previously, the cDNA was made from mRNA which encodes for light and heavy chains as well as other housekeeping genes. Therefore, after transformation of the HB101 with the puc19/cDNA plasmids, some of the transformed HB101 will contain light chain genes, some will contain heavy chain genes and some light chain genes will contain housekeeping genes. These different HB101 transformants must be sorted to get rid of those transformed HB101 harboring unwanted or housekeeping genes. The sorting process is called screening and is outlined in step 4.

4. Screening of plasmid clones (E.coli HB101 containing the puc19/cDNA plasmids) allows separation of those plasmids containing immunoglobulin genes from those plasmids containing housekeeping or unwanted genes. The HB101 harboring the plasmids are spread onto agar plates and allowed to grow into small colonies. These colonies are transferred to filters to which a radioactive probe is added. The radioactive probe is a sequence of immunoglobulin DNA which will hybridize or bind only to the plasmids containing immunmoglobulin sequences and not to plasmids containing housekeeping DNA sequences or genes. Thus, one can identify, by radioactivity, and separate those transformed HB101 containing plasmids with immunoglobulin DNA from transformed HB101 containing plasmids with housekeeping DNA. After separation of these plasmids one has populations which contain only immunoglobulin sequences. The plasmids can then be sequenced to determine the nucleotide sequence of the immunoglobulin genes.

5. Immunoglobulin DNA sequencing is performed by the Sanger dideoxy chain-termination method. Preferably this is accomplished using an automated sequence such as that supplied by Applied Biosystems, Foster City, CA. The chain termination method involves the synthesis of a DNA strand by a DNA polymerase in vitro using a single-stranded DNA template.

In detail, plasmid DNA containing cloned sequences (in this case light and heavy chain sequences) is separated into single strands by heating and quick cooling and is annealed to a synthetic oligonucleotide primer which binds adjacent to the insertion site for the cloned DNA (cDNA). Upon addition of DNA polymerase, four deoxynucleoside triphosphates, one of which is radioactive (dATP, dGTP, dCTP, dTTP) and one of four dideoxynucleotide triphosphates (ddATP, ddTTP, ddGTP, ddCTP) second strand synthesis is initiated because the primer allows DNA polymerase a starting point for replication of the single strand plasmid into a double strand plasmid. However, the primer extension or second strand synthesis is stopped before completion due to dideoxynucleoside triphosphate addition to the growing chain. Dideoxynucleotides lack a 3′ hydroxyl group and addition during DNA synthesis causes termination of the synthesis because the growing DNA requires a 3′ hydroxyl group on the deoxynucleoside being added. The entire synthesis process can be completed in as little as ten minutes. The net result of this second strand synthesis is a collection of radioactive second strands of varying lengths. Under the proper conditions, a collection of strands of varying lengths is created. To the reaction mixture is added EDTA and formamide, denatures by heating and run on electrophoresis acrylamide gels.

EXAMPLE IV

COMPUTER MODELING

Once the DNA sequence is obtained from the autoradiograph of the acrylamide gel it can be entered into the computer and analyzed and compared to other published immunoglobulin sequences using one of several software packages such as DNA Inspector II+ (Textco), IBI/Pustell (International Biotechnologies, Inc.), PC Gene from Intelligenetics, Genepro (Riverside Scientific Enterprises, Riverside, CA) and Microgenie (BloRad, Richmond, CA). Sequence information can then be used to generate three dimensional models using computer programs computer analysisusing such as the GROMOS molecular dynamics package (Aqvist, et al., (1985) J. Molec. Biol. 183:461) or FRODO: A Molecular Graphics Program (Jones, T.A. COMPUTATIONAL CRYSTALLOGRAPHY 303, Clarendon, Oxford (1982)), which are incorporated herein by reference.

With respect to the specific examples cited here in the cases of FIP and pST, the sequences of the light and heavy chain CDR regions are subjects to analysis by methods such as Hopp and Woods, Kyte and Doolittle, or Chou and Fasman, supra which are incorporated herein by reference. These methods predict the secondary and tertiary structures of the interacting heavy and light chains. This information will also allow for the establishment of an amino acid sequence (from three to twenty or more) which is conformationally structurally similar to the binding site. In the case where the conformationally structurally similar e\sequence is generated against an anti-idiotypic antibody, the sequence can be labeled and tested for binding to its corresponding idiotypic antibody. The sequence can also serve as a starting point for amino acid additions, deletions or substitutions. Substitutions can be in the form of either D or L forms of the amino acids. These next generation sequences can be further tested for binding. in this manner, an optimal sequence can be produced which duplicates the structure of the anti-id. Of course, the process can be used equally well to define the conformation of the idiotypic antibody CDR.

**Claims**

1. A method of producing an anti-idiotype antibody having a CDR which as an internal image is substantially conformationally faithful to the specific binding site of a ligand or the specific binding site of a specific binding partner of said ligand, comprising the steps of:

   a. obtaining anti-idiotype antibodies to said ligand;

   b. obtaining anti-idiotype antibodies to said specific binding partner of said ligand, said anti-idiotype antibodies having been raised in the same species as that in which said antibodies were obtained;

   c. screening the ability of said anti-idiotype antibodies to said ligand to bind to said anti-idiotype antibodies to said specific binding partner of said ligand; and

d. selecting those anti-idiotype antibodies to said ligand which are able to bind to said anti-idiotype antibodies to said specific binding partner of said ligand, whereby the CDR of the selected anti-idiotypic antibodies to said ligand is an internal image of said specific binding site of said ligand and the CDR of said anti-idiotypic antibodies to said specific binding partner of said ligand is an internal image of said specific binding site of said specific binding partner of said ligand.

2. A method of determining the conformational structure of the specific binding site of a ligand or of the specific binding site of a specific binding partner of said ligand, comprising the steps of:

a. obtaining a hybridoma cell line secreting an anti-idiotypic antibody, said anti-idiotypic antibody carrying as the CDR an internal image of said specific binding site of said ligand or of said specific binding site of said specific binding partner of said ligand;

b. isolating the mRNA of said hybridoma cell line;

c. determining the nucleotide sequence of the mRNA encoding said CDR;

d. identifying the amino acid sequence corresponding to said nucleotide sequence; and

e. generating a model of the conformational structure of a peptide comprising said amino acid sequence, whereby said model of the conformational structure of said peptide is substantially conformationally faithful to the conformational structure of the specific binding site of said ligand or said specific binding site of said specific binding partner of said ligand.

3. A method of producing a binding site mimic which is conformationally faithful to the specific binding site of a ligand or to the specific binding site of a specific binding partner or said ligand, comprising the steps of:

a. obtaining a hybridoma cell line secreting an anti-idiotypic antibody, said anti-idiotypic antibody carrying as the CDR an internal image of said binding site of said ligand or said specific binding site of said specific binding partner of said ligand;

b. isolating the mRNA of said hybridoma cell

c. determining the nucleotide sequence of the mRNA encoding said CDR;

d. identifying the amino acid sequence corresponding to said nucleotide sequence;

e. constructing a model of the conformational structure of a peptide comprising said amino acid sequence;

f. generating a binding specific mimic conformationally faithful to said conformational structure of said peptide; and

g. synthesizing said binding specific mimic.

4. The method of claim 3 wherein said binding site mimic is a peptide.

5. the method of claim 3 wherein said binding site mimic is not a peptide.

6. A binding site mimic produced by the method of claim 3.

7. A method of making a pro-binding site mimic which is conformationally suggestive of the specific binding site of a ligand or to the specific binding site of a specific binding partner of said ligand, comprising the steps of:

a. obtaining a hybridoma cell line secreting an anti-idiotypic antibody, said anti-idiotypic antibody carrying as the CDR an internal image of said specific binding site of said ligand or said specific binding site of said specific binding partner of said ligand.

b. isolating the mRNA of said hybridoma cell line;

c. determining the nucleotide sequence of the mRNA encoding said CDR;

d. identifying the amino acid sequence corresponding to said nucleotide sequence;

e. inferring the portions of said amino acid sequence likely to be on the surface when said amino acid sequence assumes a tertiary structure;

f. picking plausible amino acid or peptide chemical structures from a computer database for bridging said portions of said amino acid sequence to form a single polypeptide chain; and,

g. synthesizing a single polypeptide chain probinding site mimic having said plausible amino acid or peptide chemical structures bridging said portions of said amino acid sequence.

8. The method of claim 7, further comprising the steps of:

h. screening said single polypeptide chain probinding site mimics for specific binding affinity to said ligand or said specific binding partner of said ligand;

i. selecting those single polypeptide chain probinding site mimics having a desired specific binding affinity; and

j. synthesizing said selected single polypeptide chain pro-binding site mimics.

9. The method of claim 7, wherein said picking step further comprises the sub-steps of:

f1. creating a first group of possible amino acid or peptide chemical structures for b ridging said portions of said amino acid sequence by examining a data base of possible candidates;

15

f2. producing a second group of possible candidates by determining those candidates in the first group having a proper direction for bridging said portions;

f3. producing a third group of possible candidates by determining which of the candidates in the second group have a proper orientation for bridging said portions; and

f4. selecting from among said candidates of said third group using computer methods to fit structurally as well as possible so that said resulting single polypeptide chain pro-binding site mimic has a very high probability of folding into a conformational structure very similar to the conformational structure of said ligand or said specific binding partner of said ligand.

10. A pro-binding site mimic produced by the method of claim 7.

11. A pro-binding site mimic produced by the method of claim 8.

12. A pro-binding site mimic produced by teh method of claim 9.

13. A pro-binding site mimic produced by the method of claim 10.

14. A method of altering the conformation of a pro-binding site mimic to the specific binding site of a ligand or to the specific binding site of a specific binding partner of said ligand, comprising the steps of:

a. synthesizing a plurality of pro-binding site mimics by the method of claim 7;

b. contacting each pro-binding site mimic with the ligand of specific binding partner complementary to the ligand or specific binding partner which said pro-binding site mimic mimics;

c. detecting the degree of specific binding activity between each pro-binding site mimic an the contacted ligand or specific binding partner;

d. synthesizing a further plurality of pro-binding site mimics having systematic additions, deletions or substitutions at specific positions; and,

e. repeating steps b, c, and d until the desired degree of conformational fidelity is achieved.

15. The method of claim 15 where said desired degree of conformational fidelity is equivalent to that of the binding site mimic.

16. A method of determining the critical binding characteristics of a receptor-ligand interaction, comprising the steps of:

a. obtaining a hybridoma cell line secreting an anti-idiotype antibody, said anti-idiotype antibody carrying as the CDR an internal image of said specific binding site of said receptor;

b. isolating the mRNA of said hybridoma cell

c. determining the nucleotide sequence of the mRNA encoding said CDR;

d. identifying the amino acid sequence corresponding to said nucleotide sequence;

e. generating a computer model of the conformational structure of a peptide comprising said amino acid sequence, whereby said model of the conformational structure of said peptide is substantially conformationally faithful to the conformational structure of the receptor;

f. generating computer models of the conformational structure of a plurality of binding site mimics or pro-binding site mimics of the ligand;

g. comparing by computer means the characteristics of the binding between the modeled conformational structure of the receptor and each of the modeled conformational structures of the ligand binding site mimics or pro-binding site mimics; and,

h. determining those binding characteristics which are critical to the binding of the receptor and ligand.

17. A method designing a binding site mimic comprising a sequence of monomers, said binding site mimic having specific binding activity altered from that of the ligand which it mimics, comprising the steps of:

a. generating a binding site mimic or pro-binding site mimic of said ligand;

b. choosing one position in said monomer chain;

c. systematically modifying said binding site mimic by the addition, deletion or modification of the monomer at said chosen position;

d. contacting each modified binding site mimic with the specific binding partner, or a binding site mimic of the specific binding site of said specific binding partner of said mimicked ligand;

e. determining the binding affinity between each modified binding site mimic and said specific binding partner or binding specific mimic of said specific binding partner; and,

f. selecting one or more modified binding site mimics having the desired binding affinity.

18. The method of claim 17 comprising the further steps of:

g. electing a chosen position other than that previously chosen and repeating steps c through f one or more times.

19. A method of immunizing an animal against a pathogen, comprising the steps of:

a. synthesizing a binding site mimic corresponding to an antigenic component of said pathogen; and,

b. vaccinating said animal with said binding site mimic.

20. A method of altering the conformation of a binding site mimic to the specific binding site of a ligand or to the specific binding site of a specific binding partner of said ligand, comprising the steps of:

a. synthesizing a plurality of binding site mimics by the method of claim 7;

b. contacting each binding site mimic with the ligand of specific binding partner complementary to the ligand or specific binding partner which said binding site mimic mimics;

c. detecting the degree of specific binding activity between each binding site mimic and the contacted ligand or specific binding partner;

d. synthesizing a further plurality of binding site mimics having systematic additions, deletions or substitutions at specific positions; and,

e. repeating steps b, c and c until the desired degree of conformational fidelity is achieved.

21. A method of treating a subject suffering from a condition resulting from a lack of a particular ligand, comprising the steps of:

a. obtaining a BSM conformationally equivalent to the specific binding site of said ligand; and,

b. providing a therapeutically effective amount of said BSM in a physiologically acceptable form so as to relieve said suffering.

22. A method of detecting the presence of cells in a subject and said cells either producing or being associated with a particular ligand marker, comprising the steps of:

a. injecting said subject with a BSM conformationally equivalent to the specific binding site of said ligand marker, said BSM being conjugated to a radioisotope capable of detection; and,

b. subsequently scanning with a device to detect and locate the sites of uptake of said radiolabeled BSM.

23. A composition comprising a binding site mimic conformationally equivalent to the specific binding site of a ligand.

24. A composition comprising a BSM conformationally equivalent to the specific binding site of a ligand, said BSM being conjugated to a toxin.

25. Use of a toxin-conjugated BSM conformationally equivalent to a specific binding partner of a ligand marker on the surface of malignant cells for the preparation of a medicament for the treatment of patients having the malignant cells with the ligand marker on their surface.

26. In an immunoassay to determine the presence of a ligand in a sample of fluid, the improvement comprising employing one or more BSM's in place of one or more of the ligands or specific binding partners in the assay.

FIG. I